**Europäisches Patentamt**

**European Patent Office**

**Office européen des brevets**

(19)

(11) Veröffentlichungsnummer: **0 108 197**
**A2**

(12) # EUROPÄISCHE PATENTANMELDUNG

(21) Anmeldenummer: **83107534.6**

(22) Anmeldetag: **30.07.83**

(51) Int. Cl.³: **A 61 K 6/04**
**B 22 F 1/00**

(30) Priorität: **30.10.82 DE 3240256**

(43) Veröffentlichungstag der Anmeldung:
**16.05.84 Patentblatt 84/20**

(84) Benannte Vertragsstaaten:
**AT BE CH DE FR GB IT LI LU NL SE**

(71) Anmelder: **Blendax-Werke R. Schneider GmbH & Co.**
**Rheinallee 88**
**D-6500 Mainz(DE)**

(72) Erfinder: **Hohmann, Wolfgang, Prof. Dr.**
**Liliencronstrasse 6**
**D-6000 Frankfurt am Main 1(DE)**

(54) **Verfahren zur Herstellung von Silber-Zinn-Legierungen für zahnärztliche Amalgame.**

(57) Eine zerspante Vorlegierung für zahnärztliche Amalgame mit verbesserten Eigenschaften wird erhalten, wenn man bei der Herstellung folgende Verfahrensschritte einhält:

Verdüsen einer Silber-Zinn-Kupfer-Legierung zu einem Pulver;

Trocknen und Weiterverarbeiten dieses Pulvers durch Verpressen zu quaderförmigen Blöcken oder Rundstäben; Sintern dieser quaderförmigen Blöcke oder Rundstäbe; Zerspanen der porösen Sinterkörper.

**EP 0 108 197 A2**

Croydon Printing Company Ltd.

Verfahren zur Herstellung von Silber-Zinn-Legierungen
für zahnärztliche Amalgame

Die vorliegende Erfindung betrifft ein Verfahren zur Herstellung von Silber-Zinn-Legierungen für zahnärztliche
Amalgame mit verbesserter Stabilität und Verarbeitbarkeit.

Amalgame werden bekanntlich in weitem Umfang als Füllungsmaterialien in der Zahnheilkunde verwandt.

Die Herstellung des Amalgams geschieht in der zahnärztlichen
Praxis unmittelbar vor Applikation der Füllung durch Vermischen von Quecksilber mit silberhaltigen Vorlegierungen.

Diese Vorlegierungen bestehen in der Regel aus den Hauptbestandteilen Silber und Zinn, denen noch untergeordnete
Anteile anderer Metalle, insbesondere Kupfer, zugesetzt
sein können.

Die Struktur derartiger Legierungen entspricht überwiegend
der Zusammensetzung $Ag_3Sn$. Diese Zusammensetzung ist sehr
spröde und ergibt bei der Zerspanung durch Fräsen oder Drehen
fein zerbröckelnde Späne, die sich erforderlichenfalls noch
durch nachträgliches Mahlen weiter verkleinern lassen.

Es wird angenommen, daß sich bei der Amalgamierung ein
Teil dieses $Ag_3Sn$ ($\gamma$-Phase) zu einer Silber-Quecksilber-
Verbindung ($\gamma_1$-Phase) und einer Zinn-Quecksilber-Verbindung
($\gamma_2$-Phase) umsetzt. Die letztere entspricht etwa der Zusammensetzung $Sn_8Hg$ und ist aufgrund ihres hohen Zinnanteils
verantwortlich für die Korrosionsanfälligkeit dieses Amalgam-
Typs.

Die Korrosionsanfälligkeit kann durch Zulegierung von etwa
10 bis etwa 25 % Kupfer in diese Vorlegierung wesentlich
verringert werden, da sich aufgrund der Affinität von Kupfer
zu Zinn Kupfer/Zinn-Verbindungen wie $Cu_6Sn_5$ bzw. $Cu_3Sn$ anstelle von $Sn_8Hg$ bilden. Die daraus hergestellten Amalgame sind dann weitgehend frei von der $\gamma_2$-Phase und bei einem nicht zu niedrigen Silberanteil wesentlich korrosionsfester als die klassischen Silber/Zinn-Amalgame.

Das Zulegieren von Kupfer hat jedoch nachteilige Rückwirkungen auf die Herstellbarkeit der pulverförmigen Vorlegierungen, so daß der Vorteil des erhöhten Kupferzusatzes dadurch in Frage gestellt wird.

Um eine gute Stopfbarkeit der Legierung, eine hohe Kantenfestigkeit der Füllung und eine gewisse Toleranz in der Quecksilberdosierung zu erreichen, werden überwiegend durch Zerspanung hergestellte Legierungspulver eingesetzt. Gut zerspanbar aber sind Silber/Zinn/Kupfer-Legierungen nur in einem Bereich von etwa 40 % Silber, 30 % Zinn und 25 bis 30 % Kupfer. Hier besteht die Legierung etwa im Verhältnis 3:2 aus den spröden intermetallischen Verbindungen $Hg_3Sn$ und $Cu_3Sn$. Aus dieser Legierung hergestellte Amalgame haben deshalb einen hohen Kupfergehalt, sie erhärten im Vergleich zu klassischen Amalgamen sehr langsam und zeigen auch bei vorgeschlagenem Zinkzusatz keinen echten Silberglanz mehr.

Deshalb bestehen die heute auf dem Markt führenden Produkte weiterhin im wesentlichen aus zerspanten Silber/Zinn-Legierungen mit einem Gehalt an etwa 70 % Silber und 30 % Zinn, denen ein verdüstes Silber/Kupfer-Eutektikum zugemischt wird. Da dieses kugelförmige verdüste Pulver feiner sein muß als die Späne, beträgt die Ausbeute aus dem Verdüsungsprozeß nur etwa 25 %. Dieses verdüste Pulver selbst ist extrem korrosionsanfällig; die so hergestellten Pulvermischungen sind nur unter absolutem Luftabschluß lagerfähig. Diese Pulvermischung neigt bei Transport und Verarbeitung zur Entmischung. Darüber hinaus sind die aus diesen Pulvern hergestellten Amalgame unökonomisch, da ihr Silbergehalt wesentlich höher als erforderlich ist.

Von der Zusammensetzung her optimale Legierungen zur Herstellung von Amalgamen sind solche, die zu etwa 40 bis etwa 70, vorzugsweise 50 bis 60 %, aus Silber, etwa 10 bis etwa 25, vorzugsweise 12 bis 15 % aus Kupfer und bis maximal 30, vorzugsweise etwa 20 bis 30 % aus Zinn sowie gegebenenfalls weiteren Metallen in untergeordneten Mengen, beispielsweise bis zu maximal 2 % Zink, bestehen. Derartige Legierungen sind jedoch aufgrund ihrer Zähigkeit langspanend. Pulver können hier nur durch Verdüsung gewonnen werden. Verdüste Pulver haben jedoch im allgemeinen den zusätzlichen Nachteil der schlechten Stopfbarkeit. Ihre Verwendung erfordert sehr genaue Quecksilberdosierung und setzt eine sehr feine Körnung voraus; die Kantenfestigkeit ist schlechter als bei Füllungen aus zerspanten Legierungen.
Hier setzt nun die vorliegende Erfindung ein:

Es wurde nämlich gefunden, daß sich zur Herstellung von dentalen Amalgamen vorzüglich geeignete Legierungen mit optimalen Eigenschaften aus spanförmigen Pulvern dann herstellen lassen, wenn diese Legierungen zunächst verdüst werden, und anschließend die so gewonnenen Pulver zu einem porösen Sinterkörper weiterverarbeitet werden, aus dem dann durch Zerspanung spanförmige Sekundärpulver gewonnen werden.

Die so erhaltenen zähen spanförmigen Legierungen weisen eine hohe mechanische Festigkeit und eine überlegene Korrosionsbeständigkeit auf. Dies führt zu einer hohen Kantenfestigkeit und einer ebenfalls hohen Formstabilität ohne eine gleichzeitige Versprödung; die Pulver sind gut stopfbar und weisen gegenüber den Quecksilberbestandteilen eine ausreichende Dosierungstoleranz auf, erhärten bei der Amalgamierung schnell, besitzen hohe Lagerstabilität, entmischen sich nicht und sind darüber hinaus aufgrund der Möglichkeit, den Silbergehalt verhältnismäßig niedrig zu halten, noch überaus ökonomisch.

Das erfindungsgemäße Herstellungsverfahren wird folgendermaßen durchgeführt:

Zunächst werden die Legierungen, vorzugsweise der zuletztgenannten Zusammensetzung, verdüst. Dies kann mit den bekannten Verdüsungsverfahren geschehen, beispielsweise der bisher meistbenutzten Schutzgasverdüsung. Vorzugsweise erfolgt die Verdüsung der Ausgangslegierung jedoch mittels Preßwasserverdüsung. Die so erhaltenen Pulver werden getrocknet und in bekannter Weise zu quaderförmigen Blöcken oder Rundstäben weiterverarbeitet. Die quaderförmigen Blöcke werden vorzugsweise mechanisch, z.B. im Matrizenabziehverfahren, die Rundstäbe vorzugsweise isostatisch verpreßt.

Die so hergestellten Grünlinge werden anschließend einer
etwa halbstündigen Sinterglühung im Bereich der Rekristallisationstemperatur unter reduzierter Atmosphäre unterzogen.
Die dabei erzielte Festigkeit der erhaltenen porösen Sinterkörper ist ausreichend zur Durchführung der nachfolgenden
Zerspanung, beispielsweise mittels Drehen oder Fräsen.

Durch die hohe Porosität der Sinterkörper entstehen auch
bei prinzipiell langspanenden Legierungen kurze Bröckelspäne. Aufgrund der Spanstauchung liegen die entstandenen
Spankorngrößen deutlich unter den Ausgangspulverkorngrößen.

Die so hergestellten Späne sind durchsetzt mit feinsten,
zum Teil anhaftenden sphärischen Teilchen, was eine weitere
Qualitätsverbesserung bedeutet.

Patentansprüche

1. Verfahren zur Herstellung zerspanter Legierungen zur Herstellung zahnärztlicher Amalgame, gekennzeichnet durch die Kombination folgender Verfahrensschritte:

   Verdüsung einer Silber/Zinn/Kupferbasislegierung zu einem Pulver;

   Trocknung und Weiterverarbeitung dieses Pulvers durch Verpressen zu quaderförmigen Blöcken oder Rundstäben;

   Sinterglühung dieser quaderförmigen Blöcke oder Rundstäbe;

   Zerspanung der porösen Sinterkörper.

2. Verfahren nach Anspruch 1, dadurch gekennzeichnet, daß die Verdüsung der Vorlegierung durch Preßwasserverdüsung erfolgt.

3. Legierung zur Herstellung zahnärztlicher Amalgame, hergestellt nach dem Verfahren gemäß Anspruch 1.